# EUROPEAN PATENT SPECIFICATION

(11) **EP 1 740 166 B1**
(45) Date of publication and mention of the grant of the patent: **22.12.2010**
(21) Application number: 05741948.3
(22) Date of filing: 26.04.2005
(51) Int. Cl.: A61K 31/00

(54) **THERAPEUTIC ANTIMICROBIAL COMPOSITIONS AND METHODS**
THERAPEUTISCHE ANTIMIKROBIELLE ZUSAMMENSETZUNGEN UND METHODEN
COMPOSITIONS ANTIMICROBIENNES ET METHODES THERAPEUTIQUES

(30) Priority: 26.04.2004 US 565311 P
(43) Date of publication of application: 10.01.2007
(60) Divisional application: 10012775.2
(73) Proprietor: The Procter and Gamble Company, Cincinnati, OH 45202 (US)
(72) Inventor: LYNCH, Michael, Hollywood, FL 33020 (US); ISTVAN, Rudyard, Ft. Lauderdale, FL 33308 (US); CHERNOW, Bart, Ft. Lauderdale, FL 33305 (US); DURRANI, Manzer, Plantation, FL 33322 (US); PAN, Robert, Ya-Lin, Cincinnati, OH 45242 (US); SAUD, Abel, Loveland, OH 45140 (US); MOESE, Rosa Laura, West Chester, OH 45069 (US)
(74) Representative: Hampton, Matthew John
(86) International application number: PCT/US2005/014279
(87) International publication number: WO 2005/105070

(56) References cited:
- WO-A-2004/000016
- DE-A- 3 229 097
- A. BURGER, H. WACHTER: "Hunnius Pharmazeutisches Wörterbuch" 1993, WALTER DE GRUYTER , BERLIN, NEW YORK * page 405, paragraph DERMATIKUM *

## Description

### FIELD OF THE INVENTION

The present invention relates to therapeutic antimicrobial compositions and methods that provide enhanced immediate and residual anti-viral and antibacterial efficacy against rhinovirus, rotavirus, Gram-positive bacteria, Gram-negative bacteria and combinations thereof. More specifically, the present invention relates to antimicrobial compositions comprising an organic acid or organic acid mixture, a specific, short-chain anionic surfactant having at least one of the following: a large, hydrophilic head group; an unsaturated structure; and/or a branched structure, and to the use of said compositions for the preparation of a medicament for the treatment or prevention of diseases or infections caused by particular microbes.

### BACKGROUND OF THE INVENTION

Human and mammalian health is certainly impacted by the spread of microbial entities at home, school, work and in the environment generally. Indeed, viruses and bacteria continue to cause a variety of sicknesses and ailments, prompting high absenteeism in schools and places of employment. In the wake of widespread food poisoning and the like, the public has become even further concerned with sanitization, both of person and property. Consequently, those of skill in the art have focused their research endeavors on the identification and deployment of suitable antimicrobial compositions, and specifically those that provide immediate and residual kill of microbes, with or without the use of water.

A comprehension of the vast benefits achieved via practice of the present invention requires an understanding of the various microbes against which the present compositions are effective. Bacteria found on human skin may be divided into two groups, namely, resident and transient bacteria. Resident bacteria are Gram-positive bacteria that establish as permanent microcolonies on the surface and outermost layers of the skin. Such bacteria play a fundamental role in preventing the colonization of other, more harmful bacteria and fungi. Transient bacteria are bacteria that are not part of the normal resident of the flora of the skin. Rather, transient bacteria are deposited when airborne contaminated material lands on the skin or when contaminated material is brought into physical contact with such bacteria. Transient bacteria are typically divided into two subgroups: Gram-positive and Gram-negative. Gram-positive bacteria include pathogens such as *Staphylococcus aureus, Streptococcus pyogenes* and *Clostridium botulinum.* Gram-negative bacteria include pathogens such as *Salmonella, Escherichia coli, Klebsiella, Haemophilis, Pseudomonas aeuginosa, Proteus* and *Shigella dysenteriae.* Gram-negative bacteria are generally distinguished from Gram-positive bacteria via the existence of an additional protective cell membrane in the former, which often results in Gram-negative bacteria being less susceptible to conventional, topical antibacterial actives.

There exist several contemporary compositions and methods for reducing and/or eliminating the formation of bacteria and/or viruses. For example, it is well known that the washing of hard surfaces, food (*e.g*. fruit or vegetables) and skin, especially the hands, with antimicrobial or non-medicated soap, is effective against viruses and bacteria. Actually, removal of the viruses and bacteria is due to the surfactancy of the soap and the mechanical action of the wash procedure, rather than the function of an antimicrobial agent. Thus, it is recommended that people wash frequently to reduce the spread of viruses and bacteria. However, many conventional products and methods of sanitization, including washing, fail to address the dilemma of sanitization "on the go", that is to say, when a consumer is removed from the benefit of running water. Those skilled in the art have attempted to resolve this dilemma via the incorporation of antimicrobial agents into disinfecting lotions, cleansing wipes and the like. Such articles reduce the need for water during or following the application of the subject composition.

Other conventional antimicrobial cleansing products include deodorant soaps, hard surface cleaners, and surgical disinfectants. These traditional, rinse-off antimicrobial products have been formulated to provide bacteria removal during washing. A few such products, including antimicrobial soaps, have also been shown to provide a residual effectiveness against Gram-positive bacteria, but provide limited residual effectiveness against Gram-negative bacteria. By "residual effectiveness", it is meant that the subject antimicrobial controls microbial growth on a substrate by either preventing growth of microbes or engaging in continuous kill of microbes for some period of time following the washing and/or rinsing process. To address the dilemma of limited residual efficacy against Gram-negative bacteria, those skilled in the art have sought to incorporate high levels of alcohol and/or harsh surfactants into contemporary antimicrobial products, which have been shown to cause dryness and irritation to skin tissues.

Thus, there remains a substantial need to identify and deploy antimicrobial compositions that may be used by consumers "on the go"; provide immediate and residual kill of microbes with or without washing; and prevent dryness and irritation to skin following application. Despite providing a quasi solution to the dilemma of water availability, those skilled in the art have yet to identify antimicrobial compositions that address the problems associated with dryness and irritation to skin. In fact, attempts to resolve this dilemma have generally resulted in the adoption of aqueous-based antimicrobial formulas incorporating high levels of zwitterionic surfactants that are too weak to provide significant immediate or residual benefits. Others have attempted to address the dilemma of dryness or irritation to skin by incorporating cationic surfactants into antimicrobial compositions, which have been associated with adverse impacts on the environment and human health. Yet others still have attempted to resolve this dilemma via the incorporation of long-chain anionic surfactants into antimicrobial compositions, which are intended to prevent skin tissue penetration. Nevertheless, such surfactants are often associated with poor phase stability in product, incompatibility with commercial antimicrobial agents, and low residual kill performance. Canadian Patent No. 1,244,759 (Zerling et al.) discloses microbicidal composition which comprise a mixture of benzoic acid, methoxybenzoic acid, methylbenzoic acid, 2-furancarboxylic acid, ascorbic acid, pyruvic acid, sorbic acid or cyclohexanesulfamic acid or mixture thereof, with a C₈-C₁₈ alkyl sulfate and/or sulfonate; or a mixture of tartaric acid or glycolic acid with benzoic acid or 2-furan-carboxylic acid and a C₈-C₁₈ alkyl sulfate and/or sulfonate. The compositions are used to disinfect surfaces, especially kitchen surfaces, and as hand and skin disinfectants. The identification of a balance between the factors of antimicrobial performance, skin mildness and water availability continues to be a key concern to those of skill in the antimicrobial art.

### SUMMARY OF THE INVENTION

The present invention addresses and resolves all of the problems associated with the employment of conventional antimicrobial compositions and/or products.

In accordance with the present invention there is provided the use of an antimicrobial composition for the preparation of a medicament for the treatment or prevention of diseases or infections caused by at least one microbe selected from the group consisting of: C. Albicans, Vancomycin Resistant Enterococci (VRE), P. aeruginosa, S. marcescens, S. aureus and Methicillin Resistant Staphylococcus aureus (MRSA), characterized in that said antimicrobial composition comprises by weight:
a) from 0.2% to 70% of an organic acid; and
b) from 0.1 % to 40% of an anionic surfactant mixture having
   (i) a linear alkyl chain length of from C₄ to C₁₂ and a total head group size of at least 4 Angstroms; and/or
   (ii) a branched alkyl chain length of from C₄ to C₁₂; and/or
   (iii) a branched alkyl chain length of from C₄ to C₁₂ and a total head group size of at least 4 Angstroms;
wherein said composition is characterized by a pH of from 2.0 to 4.5;
wherein said organic acid is pyroglutamic acid; and
wherein said composition comprises C₈ alkyl glyceryl sulfonate (C8-AGS).

The antimicrobial compositions of the present invention are adapted for direct application to human skin, without causing dryness or irritation. Moreover, the antimicrobial compositions of the present invention are designed for use with or without water, and provide immediate and residual effectiveness in either instance against the recited microbes.

The specific, anionic surfactant of the present invention presents a particularly novel aspect of the present compositions. Those of skill in the art have generally relied upon the incorporation of longer chain (i.e. C₁₂ to C₁₆) anionic surfactants into antimicrobial compositions. Conventional surfactants, comparable to the acyl components found in the phospholipid matrix of the cell membrane of bacteria and virus, are thought to possess optimum antimicrobial activity with reduced skin tissue penetration. However, conventional anionic surfactants have been associated with low solubility under acidic conditions, poor compatibility with cationic antimicrobial agents, slow dissolution kinetics and limited residual antimicrobial performance.

Against the conventional wisdom in the art, the short chain anionic surfactants of the present invention comprise at least one of the following characteristics: a large, hydrophilic head group; an unsaturated structure; and/or a branched structure. Indeed, the surfactants of the present invention have traditionally been regarded as unsuitable for incorporation into an antimicrobial composition, based on the belief that such surfactants possess relatively low surface activity. Contrary to the traditional wisdom, it has been surprisingly discovered that the surfactants of the present invention deliver enhanced antimicrobial efficacy against the recited microbes. More importantly, the large head group, unsaturated structure and/or branched structure of the present surfactants reduces or limits their tendency to penetrate skin tissue, while maximizing the immediate and residual effectiveness of the antimicrobial compositions in which they are incorporated. Further, the anionic surfactants of the present invention exhibit stability in an aqueous product at a low pH, are compatible with cationic antimicrobial agents and convey strong residual antimicrobial activity when the substrate on which they are applied is later inoculated with virus or bacteria.

Antimicrobial compositions that provide enhanced immediate and residual anti-viral and antibacterial efficacy against rhinovirus, rotavirus, Gram-positive bacteria, Gram-negative bacteria and combinations thereof are described in commonly-assigned United States patent publications 20030235550 A1, 20040001797 A1, and in published PCT application WO 2004/000016. These antimicrobial compositions comprise an organic acid or organic acid mixture, a specific short-chain anionic surfactant with branching or a large head group, and, optionally, a calcium ion scavenger and/or anti-foam agents.

In another aspect of the invention the antimicrobial compositions disclosed herein optionally further comprise a calcium ion scavenger and/or an anti-foam agent.

These and other objects, features, and advantages will become apparent to those of ordinary skill in the art from a reading of the following detailed description and the appended claims. All percentages, ratios and proportions herein are by weight, unless otherwise specified. All temperatures are in degrees Celsius (° C) unless otherwise specified.

### DETAILED DESCRIPTION OF THE INVENTION

Antimicrobial compositions that provide enhanced immediate and residual anti-viral and antibacterial efficacy against rhinovirus, rotavirus, Gram-positive bacteria, Gram-negative bacteria and combinations thereof are described in commonly-assigned United States patent publications 20030235550 A1, 20040001797A1, and in published PCT application WO 2004/000016. These compositions were stated to have efficacy against gram negative and gram positive bacteria and also against viruses. We believe that certain formulations of these short chain, big head surfactant based antimicrobial compositions will be effective against a wide variety of pathenogenic agricultural organisms including but not limited to citrus canker.

These antimicrobial compositions comprise an organic acid or organic acid mixture, a specific short-chain anionic surfactant with branching or a large head group, and, optionally, a calcium ion scavenger and/or anti-foam agents. For therapeutic use the formulations require in addition to the surfactant, an organic acid and a nonionic agent. Preferred is to use C8-AGS as the surfactant, pyrrolidone carboxylic acid as the organic acid and ethylhexyl glycerol ether (EHOP) as the non-ionic agent. One of skill in the art will recognize that other agents as disclosed in 20030235550 A1, 20040001797A1, or WO 2004/000016 may be substituted. Preferred are compositions having greater than about 0.50% C8AGS with most preferred compositions containing at least 1.25% C8AGS and a pyrrolidone carboxylic acid content of greater than or equal to 2.0%.

One of skill in the art will readily recognize that other active ingredients can be included to provide different properties or to improve the effectiveness of the present compositions against specific, organisms. When adding other actives, the concentration of surfactants, acids and/or non-ionic agents would also be modified to provide the degree of activity desired. For example, Parachlorometaxylenol (PCMX), a known antimicrobial may be added to provide another broad spectrum antimicrobial. For example when adding PCMX, it is possible to reduce the amount of C8AGS below 1% concentration by weight.

Five formulations of antimicrobial compositions were created having the active agents set forth in Table 1. Water makes up the balance of the formulation. These formulations were made in accordance with 20030235550 A1, 20040001797A1, and in published PCT application WO 2004/000016.

**Table 1.**

| | **Formula** | | | | |
|---|---|---|---|---|---|
| **Active Component (Wt %)** | **R*** | **M** | **H** | **KS** | **KSM** |
| C8-AGS | 0.50 | 1.25 | 2.00 | 0.60 | 1.00 |
| Coco Sulfofatty Acid | | | | 0.40 | |
| Gluconic Acid | 2.00 | | | | |
| Pyroglutamic Acid (PCA) | | 2.00 | 15.00 | 3.50 | 3.00 |
| Succinic Acid | | | | 1.50 | 2.00 |
| Ethylhexyl Glyceryl Ether | | | | | |
| (EHOP) | | 0.55 | 1.00 | 0.50 | 0.50 |
| Parachlorometaxylenol | | | | | |
| (PCMX) | | | | 0.35 | 0.50 |
| Potassium Sorbate | | | | 0.10 | |

| | | | | | |
|---|---|---|---|---|---|
| * Comparative | | | | | |

### Formulation

The composition of the present invention may be formulated for use in any manner known to one of skill in the art. Formulations for topical, mucosal and aerosol delivery of drugs are taught in Modern Pharmaceutics by Gilbert S. Banker (Editor), Christopher T. Rhodes (Editor) Marcel Dekker, 4th edition (June 15, 2002) ISBN: 0824706749. Reference is also made to the International Journal of Pharmaceutical Compounding which can be accessed at www.ijpc.com. These sources teach and describe the basics of pharmaceutical compounding. One of skill in the art will know how to take the active ingredients of the present invention and formulate them for delivery. Such formulations may take the form of lotions, ointments, gels, creams, drops washes, pastes, suppositories, lozenges, mouthwashes, gargles, douches, foams, surface coatings, liposomes, microspheres and transdermal patches.

One of skill in the art will appreciate that the activity of the present compositions can be affected through the selection of excipients to provide varying degree of skin penetration or to control release. Activity of the present formulations can be increased by occlusion of the skin after application with a suitable bandage or wrap. One of skill in the art will also recognize that persistent action can be increased by use of controlled release technologies which delay release of active over time.

The formulations contemplated herein can also be coated or otherwise incorporated into medical devices such as wipes, sponges, bandages, surgical drapes, hospital gowns, surgical gowns. Formulations can be developed that are suitable for disinfecting medical devices. Such formulations could be in the form of a liquid which could be used for spraying onto surfaces, soaking of devices, pumping through devices or incorporated into wipes for decontaminating a surface.

### Testing

The formulations described above were tested for Minimum Bactericidal Dilution and Residual efficacy against *C. Albicans, E. Coli, S. marcesens, Methicillin Resistant Staphylococcus aureus,* and *E. faecalis,*

### Minimum Bactericidal Dilution.

Various formulations as set out in Table 2 below were tested for Minimum Bactericidal Dilution. Organisms to be tested were grown on slants and transferred to an agar plate by streaking to form a lawn. Colonies are scraped off the agar plates using a sterile innoculating loop and suspended in phosphate buffered solution (PBS) and diluted to 5 x 10⁶ CFU/ml.

**Table 2**

| | | **MINIMUM BACTERICIDAL DILUTION** | | | | | | |
|---|---|---|---|---|---|---|---|---|
| **HTR #** | **Test Article** | **C. albicans 10231** | **faecalis is (VRE) 51299** | **E. coli 11229** | **P. aeruginosa 15442** | **S. marcescens 14756** | **S. aureus 6538** | **S. aureus (MRSA) 33591** |
| 1 | **0.1%** EHOP, 1.25% AGS, 8.5% PCA, | No Inhibition | 1:8 | 1:8 | 1:16 | 1:2 | Undiluted | Undiluted |
| 2 | 1 % EHOP, 0.5% AGS, 2% PCA | No Inhibition | 1:2 | 1:16 | 1:8 | 1:4 | No Inhibition | Undiluted |
| 3 | 0.1% EHOP, 2% AGS, 15% PCA | No Inhibition | 1:4 | 1:16 | 1:16 | 1:4 | 1:2 | 1:2 |
| 4 | 0.1% EHOP, 0.5% AGS, PCA | No Inhibition 15% | Undiluted | Undiluted | 1:2 | 1:8 | Undiluted | 1:4 |
| 5 | 0.1% EHOP, 2% AGS, 2% PCA | No Inhibition | 1:4 | 1:2 | 1:8 | 1:8 | 1:2 | 1:8 |
| 6 | 0.55% EHOP, 1.25% AGS 15% PCA | No Inhibition | 1:32 | 1:4 | 1:1024 | 1:8 | Undiluted | 1:16 |
| | | | | | 1:4 | | | |
| | | | | | 1:4 | | | |
| 7 | 0.1% EHOP, 0.5% AGS, 2% PCA | No Inhibition | Undiluted | 1:8 | 1:8 | Undiluted | No Inhibition | No Inhibition |
| 8 | 1% EHOP, 2% AGS, 15% PCA | Undiluted | 1:32 | 1:32 | 1:64 | 1:64 | 1:16 | 1:2 |
| 9 | 1% EHOP, 2% AGS, 2% | No Inhibition PCA | 1:16 | 1:16 | 1:8 | 1:8 | 1:16 | 1:16 |
| 10 | 0.55% EHOP, 2% AGS, 8.5% PCA | Undiluted | 1:16 | 1:32 | 1:4 | 1:2 | 1:4 | 1:8 |
| 11 | 1% EHOP, 1.25% AGS, 8.5% PCA | Undiluted | Undiluted | 1:2 | 1:4 | 1:4 | 1:4 | 1:8 |
| 12 | 0.55% EHOP, 1.25% AGS, 8.5% PCA | Undiluted | 1:2 | 1:8 | 1:16 | 1:2 | 1:4 | 1:8 |
| | | | | | 1:4 | | | |
| | | | | | 1:2 | | | |
| 13 | 0.55% EHOP, 1.25% AGS, PCA | No Inhibition 2% | 1:16 | 1:8 | 1:4 | 1:4 | 1:4 | 1:4 |
| 14 | 0.55% EHOP, 0.5% AGS, 8.5% PCA | No Inhibition | 1:2 | Undiluted | 1:8 | 1:4 | Undiluted | Undiluted |
| 15 | 1% EHOP, 0.5% AGS, 15% PCA | No inhibition | Undiluted | Undiluted | 1:4 | 1:16 | Undiluted | Undiluted |
| 16 | 0.55% EHOP, 1.25% AGS, 8.5% PCA | No Inhibition | 1:8 | 1:16 | 1:32 | 11:32 | Undiluted 1:8 | 1:2 Undiluted |
| | | | | | 1:4 | | | |
| | | | | | Undiluted | | | |
| 17 | 0.55% EHOP, 1.25% AGS, 8.5% PCA | No Inhibition | 1:32 | 1:8 | 1:2048 | | | |
| | | | | | 1:4 | | | |
| | | | | | 1:8 | | | |
| 18 | 0.55% EHOP, 1.25% AGS, 8.5% PCA | No | 1:256 | 1:16 | 1:32 | 1:8 | 1:4 | 1:8 |
| | | | 1:2 | | 1:4 | | | |
| | | | Inhibition 1:2 | | 1:4 | | | |
| 19 | 0.55% EHOP, 1.25% AGS, 8.5% PCA | No Inhibition | 1:8 | 1:4 | 1:256 | 1:2 | Undiluted | 1:2 |
| | | | | 1:2 | 1:4 | | | |
| | | | | 1:2 | 1:4 | | | |
| 20 | 0.55% EHOP, 1.25% AGS, 8.5% PCA | No Inhibition | 1:16 | 1:32 | 1:2048 | 1:2 | Undiluted | 1:2 |
| | | | | | 1:16 | | | |
| | | | | | 1:4 | | | |
| 21 | ChloraPrep | 1:4 | 1:4 | 1:64 | 1:2 | 1:2 | 1:32 | 1:8 |

### In Vitro Time Kill

Candida Albicans was cultured under appropriate conditions. (current USP procedures for culturing organisms is appropriate.) Incubation period varies (typically 120 hours at 25°C +/- 1°C) to a density of between 1.0E+06 - 1.0E+07 CFUs/mL. Actual CFUs/mL of starting cultures were determined by serially diluting and plating an aliquot (typically plate 10⁻⁶, 10⁻⁵, 10⁻⁴, 10⁻³, 10⁻² dilutions). A 50µl aliquot of the yeast culture was pipetted into 5.0mL of the antimicrobial solution(s) in a sterile scintillation vial (organisms: solution ratio = 1:100) and mixed thoroughly by vortexing. Test inoculum level was ~ 1.0E+04-1.0E+05 CFUs/mL. At the predetermined time point(s), 1 min, 5 min, and 10 min, a 0.5mL aliquot of the inoculated antimicrobial solution is pipetted into 4.5mL of Dey/Engley (D/E) neutralizing broth (ratio = 1:10) and mixed by vortexing. An aliquot of the neutralized sample including yeast was plated onto a Sabouaud Dextrose Agar (SDA) plate using standard pour plate techniques. The SDA plates were incubated at 25°C+/- 1°C for 5 days (~120 hours) and CFU's are counted. CFUs/mL for organism cultures are calculated and compared to CFUs/mL for antimicrobial solutions to determine the log reductions.

### Residual Skin Testing

Residual skin testing was performed by evenly coating the surface of a skin patch with 20µl of the active solution. Skin samples were allowed to evaporate for 1 minute, 15 minutes , 60 minutes, 120 minutes, 240 minutes,360 minutes, 480 minutes, and 14 hours with the lid off the Petri plate. At the appropriate time point, skin samples were inoculated with 10µl of a 1:10 dilution of the 18-hour microbial suspension (~1.0E+08 CFUs/mL), evenly covering the entire area and the sample recovered and allowed to sit 5 minutes. At this time the skin was extracted using sterile forceps and placed in a steril centrifuge tube. Containing 10ml of a sampling solution and vortexed for 30 seconds. An aliquot of the extracted sample containing any microbials from the skin was plated onto a trypticase soy agar plate using a spiral plater (typically 50µl in exponential mode). The agar plates were incubated at 37°C overnight (~18 hours) and CFUs are counted.CFUs/mL established by Baseline Count are calculated and compared to CFUs/mL for antimicrobial/bacterial solutions to determine the log reductions. The Baseline Count was achieved by evenly spreading 10µl of the diluted bacterial suspension on a square of the Skin and processed in accordance with the above procedure, except no active solution was added.

### Test results

### Anti-Fungal/Yeast Activity

Formulas R, M, H, KS and KSM were tested against Candida albicans and compared for activity with Chloraprep. The antifungal properties of these formulations were previously untested arid unknown. In vitro time kill testing was performed as described above. The result of the testing are set forth in Table 3.

**Table 3**

| In vitro time-kill of C. albicans 10231 | | | |
|---|---|---|---|
| | Minutes of contact time (log reduction from log 5.0 titer) | | |
| Formula sample | 1 | 5 | 10 |
| ChloraPrep (70% alcohol + 2% CHG) | 4.8 | 4.8 | 4.8 |
| KSM | 4.8 | 4.8 | 4.8 |
| M | 1.3 | 4.8 | 4.8 |
| RID | 0.3 | 0.9 | 1.0 |

Residual Skin efficacy testing against C. albicans was performed as set forth above. The data are set forth in Table 4 below.

**Table 4 Candida albicans (C. albicans 10231) Residual Efficacy**

| **Log Reduction** | **Chlora-prep** | **RID** | **M** | **H** | **KS** | **KSM** |
|---|---|---|---|---|---|---|
| 1 min | 3.6 | 0.8 | 2.3 | 3.4 | 3.6 | 3.6 |
| 15 min | 2.2 | 1.1 | 2.7 | 3.9 | 3.6 | 3.6 |
| 60 min | 2.5 | 0.9 | 2.9 | 3.9 | 3.6 | 3.6 |
| 120 min | 1.8 | 2.1 | 3.2 | 3.9 | 3.6 | 3.6 |
| 240 min | 1.1 | 0.2 | 2.7 | 3.9 | 3.6 | 3.6 |

The above data show that medically acceptable strengths of RID kill candida. Faster kill, but not more residual activity can be obtained by adding known broad spectrum antimicrobial (PCMX).

### E.Coli

Formulas R, M, H, KS and KSM were tested against E. coli and compared with Chloraprep. Residual Skin efficacy testing against E.Coli was performed as set forth above. The data are set forth in Table 5 below.

**Table 5 E. coli (E. coli 11229) Residual efficacy testing.**

| **Log Reduction** | **Chlora- prep** | **RID** | **M** | **H** | **KS** | **KSM** |
|---|---|---|---|---|---|---|
| 1 min | 4.6 | 0.6 | 4.6 | 4.6 | 4.6 | 4.6 |
| 15 min | 4.5 | 2.7 | 4.6 | 4.6 | 3.7 | 4.5 |
| 60 min | 4.1 | 4.5 | 4.6 | 4.6 | 4.6 | 4.6 |
| 120 min | 3.3 | 3.7 | 4.6 | 4.2 | 4.6 | 4.6 |
| 240 min | 3.9 | 4.0 | 4.6 | 4.1 | 4.6 | 4.6 |

### Serratia marcesens (S. marcesens 14756)

Formulas R, M, H, KS and KSM were tested against S. marcesens and compared with Chloraprep. Residual Skin efficacy testing against S. marcesens was performed as set forth above. The data are set forth in Table 6 below.

**Table 6 S. marcesens 14756 Residual Efficacy Results**

| **Log Reduction** | **Chlora-prep** | **RID** | **M** | **H** | **KS** | **KSM** |
|---|---|---|---|---|---|---|
| 1 min | 1.8 | 0.1 | 3.6 | 6.0 | 5.1 | 5.1 |
| 15 min | 0.7 | 0.1 | 3.6 | 6.0 | 5.1 | 5.1 |
| 60 min | 2.0 | 0.9 | 2.0 | 6.0 | 4.5 | 5.1 |
| 120 min | 1.1 | 0.0 | 3.7 | 6.0 | 5.1 | 5.1 |
| 240 min | 1.0 | 0.2 | 2.8 | 6.0 | 5.1 | 5.1 |

### Methicillin Resistant Staphylococcus aureus (MRSA)

Formulas R, M, H, KS and KSM were tested against *MRSA* and compared with Chloraprep. Residual Skin efficacy testing against *MRSA* was performed as set forth above. The data are set forth in Table 7 below.

**Table 7 MRSA (S. aureus 33591) Residual Efficacy Results**

| **Log Reduction** | **Chlora- prep** | **R** | **M** | **H** | **KS** | **KSM** |
|---|---|---|---|---|---|---|
| 1 min | 5.5 | 1.3 | 0.9 | 4.9 | 5.1 | 5.1 |
| 15 min | 1.4 | 0.2 | 0.6 | 3.9 | 5.0 | 4.9 |
| 60 min | 2.7 | 0.1 | 0.6 | 2.9 | 5.8 | 4.9 |
| 120 min | 3.9 | 0.3 | 1.3 | 4.0 | 4.8 | 4.5 |
| 240 min | 3.7 | 0.4 | 1.7 | 4.6 | 5.0 | 4.8 |

### Vancomycin Resistant Enterococci (VRE)

Formulas R, M, H, KS and KSM were tested against E. faecalis and compared with Chloraprep. Residual Skin efficacy testing against E. faecalis was performed as set forth above. The data are set forth in Table 8 below.

**Table 8VRE (E. faecalis 51299) Residual Efficacy Results**

| **Log Reduction** | **Chlora-prep** | **RID** | **GMP / M** | **GMP / H** | **GMP / KS** | **GMP / KSM** |
|---|---|---|---|---|---|---|
| 1 min | 4.3 | 0.5 | 4.7 | 4.7 | 4.8 | 4.8 |
| 15 min | 1.1 | 1.3 | 4.5 | 4.7 | 4.8 | 4.4 |
| 60 min | 1.5 | 2.0 | 4.7 | 4.7 | 4.8 | 4.8 |
| 120 min | 1.7 | 2.8 | 4.7 | 4.7 | 4.6 | 4.8 |
| 240 min | 3.2 | 0.6 | 4.7 | 4.7 | 4.8 | 4.8 |

### Therapeutic examples

### Example 1 Acne

Acne is a common disease affecting millions of Americans every year. The condition is characterized by lesions or pimples caused by infection with P. acnes and/or P. granulosum bacteria. Common treatment is with topical retinoids, steroids, antibiotics, and topical counterirritants such as sulfur, benzoyl peroxide and/or alcohol and/or use of systemic antibiotics.

Acne could be treated and its spread prevented by topical administration of the formulations disclosed herein in a bar, pledget, lotion, gel, or cream.

### Example 2 Atopic dermatits

Atopic dermatits is a condition affecting 3-10% of the US. It most commonly affects young children and is characterized by scaly patches, small vescicles, excoriations, crusting, impetiginization caused by the presence of S. aureus in the horny layer of epidermis. Current treatment is with emollients, topical corticosteroids and/or topical or oral antihistamines. Severe cases may also be treated with UV-B phototherapy, psoralen + UVA, oral corticosteroids.

Antimicrobial compositions of the type disclosed herein have been shown to be useful in killing S. aureus bacteria. Atopic dermatits could be treated and its spread prevented through formulations of the present invention for topical administration in a soap, pledget, lotion, gel, or cream.

### Example 3 Blepharitis

Blepharitis causes lifelong chronic inflammation of eyelids is caused by Staphylococcus or yeast colonization on eyelid margins. Transmission is possible by touch. Current treatment includes use of warm compresses and lid scrubs. See, Harrison's Principals of Internal Medicine, 15th Edition.

Antimicrobial compositions of the type disclosed herein have been shown to be useful in killing S. aureus bacteria. Blepharitis could be treated and its spread prevented through formulations of the present invention for topical administration in ointments, drops, or other fomulations suitable for ocular administration.

### Example 4 Diaper Rash/Dermatitis

Diaper rash is caused by irritation from contact with urine and stool that leads to redness and breakdown of skin. Frequently accompanying common diaper rash is diaper dermatitis. Diaper dermatitis is most frequently caused by strains of the fungus Candida. Secondary bacterial infections of staphylococcus and enterobacter are also possible. Current treatments include zinc oxide cream and topical antifungal agents. It is believe that 20% of infants within first 2 years Only 10% of cases are reported. See Cecil Textbook of Medicine 22nd Edition 2004.

Treatment and/or prevention of the primary candida infection should be possible by use of the present formulations in by application. Antimicrobial compositions of the type disclosed herein have been shown to be useful in killing candida, S. aureus and enterobacter bacteria. Diaper rash and dermatitis could be treated and its spread prevented through formulations of the present invention for topical administration in a bar, pledget, lotion, gel, ointment or cream.

### Example 5 Fungal skin infections

A variety of skin infections are cause by fungal infection Trichophyton rubrum, Trichophyton mentagrophytes, Epidennophyton floccosum, demratophytes, yeasts and molds. Athlete's foot (tinea pedis) and nail fungus being among the more common. Athletes foot is commonly treated with antifungal creams, sprays and powders, oral medications. Nail fungus and toenail fungus in particular are more difficult to treat due to the protective nail. Common OTC nail creams and ointments are relatively ineffective. Oral antifungals such as itraconazole offer good efficacy but are expensive. See Cecil Textbook of Medicine 22nd Edition 2004.

Fungal skin infections could be treated and its spread prevented through formulations of the present invention for topical administration in a bar, pledget, lotion, gel, ointment or cream.

### Example 6 Impetigo

Impetigo is the most common bacterial skin infection in children. This disease is characterized by various skin lesions, including bulla, vesicles and blisters caused by S. aureus and/or Streptococcus. Impetigo contagiosa is the most common form, first appearing as blisters or red sores. Eventually, the blisters rupture and ooze a fluid that forms a crust over the affected area. The sores may tend to itch, but generally do not cause pain although there may be swollen lymph nodes in the affected area. Impetigo contagiosa is highly contagious and is readily spread to other parts of the body through touching or scratching the sores. It is also readily spread to others through personal contact and by sharing items with an infected person.

Impetigo contagiosa is readily treated through improved hygiene and topical antiobiotic ointments and/or oral antibiotics. In most cases, healing begins within three days of treatment. Though it is seldom serious, this infection can sometimes lead to complications, such as scarring, if left untreated. See Cecil Textbook of Medicine 22nd Edition 2004 and MayoClinic.com.

We believe that formulations of the present invention will treat and prevent impetigo by killing and stopping the transfer of S. aureus and other bacteria. Impetigo could be treated and its spread prevented through formulations of the present invention for topical administration in a bar, pledget, lotion, gel, ointment or cream.

### Example 7 Thrush

Oropharyngeal candidiasis (OPC) or thrush is a candida fungal infection of the mouth, throat and/or tongue. It is common in newborns, infants, and older adults, but can occur at any age. Thrush due to T-cell dysfunction is the most common opportunistic infection in HIV affecting 80-90% of all patients with advanced stages of HIV infection. Thrush is commonly treated with nonprescription and prescription antifungals and in HIV infected patients through the concommitant use of oral azoles, and possibly intrvenous amphotericin in severe refractory cases. See Cecil Textbook of Medicine 22nd Edition 2004.

Thrush can be difficult to treat in infants due to the many places that candida species can hide. Reinfection may occur from toys, pacifiers, mother's breast, etc. Because of the fast rate at which yeast multiply, Current therapies includ treatment with nystatin 4-8 or more times per day. The residual action of the present compositions should reduce frequency of application and improve rate of recovery by reducing ability of candida to hide and spread. Preferential application is topical application to mother's breasts, infant pacifiers and to swab affected areas of the mouth. Adults may also be treated in the same manner as well as with mouthwashes and gargles.

### Example 8 Trachoma

Trachoma is chronic conjuctival inflammation cause by C. trachomatis. It is common in developing world countries and is the cause of at least 4 million cases of blindness and 5 million cases of low vision. Current treatment is through surgury of the eyelid and topical antibiotics. See Cecil Textbook of Medicine 22nd Edition 2004.

Formulations of the present invention can be applied as drops or ointment to the eyes to treat and/or prevent spread of trachoma.

### Example 9 Warts

Warts are hard growths on skin cause by the Human papillomavirus. The primary treatment means is topical treatmentwith salicylic acid. See Cecil Textbook of Medicine 22nd Edition 2004.

Warts could be treated with the antiviral compositions of the present application through any topical formulation and/or use of treated bandages.

### Example 10 Avian Flu/Influenza

Avian flu is a highly infections and frequently fatal virus caused by Influenza A.. Its fatality rate is 33% vs. 10% for SARS. Treatment consists of destroying infected birds and decontaminating tools and clothing that may have come in contact with the virus using bleach solutions. See the World Health Organization avian influenza fact sheet 2004.

Formulations of the present invention could be administered to affected livestock via sprays to treat and prevent the spread of outbreaks. The present invention can also be used for decontamination of skin, clothing and tools by topical application, spray or wipes.

Formulations of the present invention can also be used to kill SARS, HBV, HPV, HIV and

### Example 11 Foot and mouth disease

Foot and mouth disease is a rapidly spread aphthovirus infection of cattle and swine that causes fever and blistering. Treatement is by culling and disposing of infected animals. Formulations of the present invention could be topically applied to animals, people and related materials and tools to prevent the spread of the virus.

### Example 12 Mastitis

Bacterial infection cause inflammation, tenderness and pain in the udders of cows caused by S. agalactia, S. aureus, and/or mycoplasma. If untreated, mastitis can lead to destruction of the udder, lost productivity and infected milk. Current treatments include discinfecting teats with iodine after milking and culling animals with chronic cases, and treatment with topical and systemic antibiotics. Hygiene plays a significant role in preventing the disease. See the Merck Veteminary Manual, Eighth Edition, Forbes, January 1998; The Knoxville News-Sentinel, November 2002; UGA Animal & Dairy Science Annual Report 1995, Analysis of Mastitis Costs (Eberhart et al., 1987); Animal Pham Reports - Mastitis: Challenges and Opportunities, 2002 (Executive Summary). Formulations of the present invention can be used to topically treat the udder.

### Example 13 Chemical sterilization

Provide alternative to existing chemical sterilants, which cause safety concerns for healthcare lab workers and corrosion of instruments with motors, moving parts and nonmetal (e.g., plastic) interfaces. Devices to be sterilized could be soaked, washed, wiped, sprayed or flushed with compositions of the present invention. Depending on the application, it may be necessary to use a sterile water or saline flush to remove any residual actives. Potential uses include any surface or device needing sterilization including but not limited to analytical equipment, robotic devices, vital signs monitoring lead wires and instruments, etc.

### Example 14 Impregnated or coated patient care or personal hygiene devices

Many medical devices or personal care products are impregnated with chlorhexidine gluocnate (CHG). CHG has been shown to cause hypersensitivity reactions when used in intravenous catheters, topical antimicrobial skin dressings, and implanted antimicrobial surgical mesh. Formulations of the present invention are believed to be ultra mild and benign to the skin. Therefore, formulations of the present invention could be used to create an active barrier on commonly used devices in contact with patients. Any medical device in contact with a patient can be potentially coated with the present formulations. For example, ventilator infections affect 35% of all mechanically-ventilated patients, are associated with 33%-35% mortality, and increase stays in ICUs by an average of 13 days. Ventilator tubing could be treated with formulations of the present invention to reduce or eliminate source of infection. Similarly, ECG lead sets and other reusable.

### Example 15 Personal Care products

Formulations of the present invention can be impregnated or coated on any personal care product in contact with skin or mucous membrane. Potential uses include device needing sterilization including but not limited to tampons and sanitary napkins, paper towels, toilet tissue, diapers, bandages, clothing, bed sheets, masks, surgical drapes, etc. For example, impregnated tampons could actively inhibit growth of group A streptococcal bacteria that cause toxic shock syndrome.

### Example 16 Condom coating

Formulations of the present invention are also suitable for coating condoms to prevent spread of sexually transmitted bacterial viral or fungal diseases, including but not limited to gonnorhea, syphilis, HIV, and HPV. Such formulations could be added during manufacturing or incorporated into lubricating gels or lotions for application during use. Care must be taken to choose excipients that are not damaging to the condom materials.

### Example 17 Feminine hygiene products

Products of the present invention can also be formulated as a douch, gel, lotion or foam to treat or prevent urinary tract infections ("UTIs"), toxic shock and prevent the spread of sexually transmitted diseases. The most common cause of UTIs is E. coli bacteria. Using diaphragms, spermicide-coated condoms, and in-dwelling catheters can increase the risk of UTIs. Women often develop a UTI when they become sexually active because the bacteria from the vaginal area may be pushed into the urethra. Formulations of the present invention can be coated on diaphrams, sponges prior to insertion. Formulations in the form of a douche or wash can be used to cleanse the vaginal canal. Formulations of the present invention could be packaged in individual dosage forms for use immediately prior to sexual contact and could include dosage forms suitable for application to diaphram or condom and suitable for disinfecting skin.

### Example 18 Otic solutions

Formulations of the present invention can be formulated for use in the ears to prevent or treat swimmers ear, and microbial ear infections.

### Example 19 Body washes and Shampoos

Formulations of the present inventions can be incorporated into therapeutic shampoos for use in humans or animals to treat diseases of the skin caused by microbial organisms.

To the extent that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the scope of the invention.

## Claims

1. Use of an antimicrobial composition for the preparation of a medicament for the treatment or prevention of diseases or infections caused by at least one microbe selected from the group consisting of: C. Albicans, Vancomycin Resistant Enterococci (VRE), P. aeruginosa, S. marcescens, S. aureus and Methicillin Resistant Staphylococcus aureus (MRSA), **characterized in that** said antimicrobial composition comprises by weight:
a) from 0.2% to 70% of an organic acid; and
b) from 0.1 % to 40% of an anionic surfactant mixture having
(i) a linear alkyl chain length of from C₄ to C₁₂ and a total head group size of at least 4 Angstroms; and/or
(ii) a branched alkyl chain length of from C₄ to C₁₂; and/or
(iii) a branched alkyl chain length of from C₄ to C₁₂ and a total head group size of at least 4 Angstroms;
wherein said composition is **characterized by** a pH of from 20 to 4.5;
wherein said organic acid is pyroglutamic acid; and
wherein said composition comprises C₈ alkyl glyceryl sulfonate (C8-AGS).

2. The use according to claim 1, wherein said diseases or infections comprise: atopic dermatitis, diaper dermatitis, impetigo, blepharitis, mastitis and oropharyngeal candidiasis (OPC).

3. The use according to claim 1, wherein said antimicrobial composition comprises at least 0.5% C8-alkyl glyceryl sulfonate (C8-AGS).

4. The use according to any of the preceding claims, wherein the antimicrobial composition further comprises a nonionic agent.

5. The use according to claim 4, wherein said nonionic agent is selected from the group consisting of: 1-(2-ethylhexyl) glycerol ether, octyl glycerol ether, 2-(2-ethylhexylxoxy) octyloxy-propanol, 1-(2-ethylhexyloxy) ethanol, octyloxy ethanol, 1,2-hexylenediol, 1,2-cyclohexanedimethanol, isopropyl glycerol ether and combinations thereof.

6. The use according to claim 5, wherein said nonionic agent comprises 1-(2-ethylhexyl)glycerol ether.

7. The use according to any of claims 4 to 6, wherein said nonionic agent is present in said antimicrobial composition in an amount of 0.1% to 10% by weight of total composition.

8. The use according to claim 1, wherein said antimicrobial composition comprises:
at least 2.0% by weight of total composition of pyroglutamic acid; and
at least 1.25% by weight of total composition of the anionic surfactant C₈ alkyl glyceryl sulfonate.

9. The use according to any one of claims 1 to 8, wherein said medicament is applied typically.

10. The use according to any one of claims 1 to 9, wherein the antimicrobial composition is applied using a wash, spray, soak, or a wipe.

11. The use according to any one of claims 1 to 10, wherein said medicament is applied as an impregnate or coating of a personal care product.

12. The use according to any one of claims 1 to 10, wherein said composition is applied in an effective amount to disinfect a medical device for use on a patient.

## Patentansprüche

1. Verwendung einer antimikrobiellen Zusammensetzung zur Herstellung eines Medikaments für die Behandlung oder Prävention von Krankheiten oder Infektionen, die durch wenigstens eine Mikrobe verursacht sind, die aus der Gruppe ausgewählt ist, die aus *C. albicans,* Vancomycinresistenten Enterokokken (VRE), *P. aeruginosa, S. marcescens, S. aureus* und Methicillin-resistentem *Staphylococcus aureus* (MRSA) besteht, **dadurch gekennzeichnet, dass** die antimikrobielle Zusammensetzung Folgendes umfasst:
a) 0,2 bis 70 Gew.-% einer organischen Säure; und
b) 0,1 bis 40 Gew.-% eines Gemischs anionischer Tenside mit
(i) einer Länge einer linearen Alkylkette von C₄ bis C₁₂ und einer Gesamtkopfgruppengröße von wenigstens 4 angström; und/oder
(ii) einer Länge einer verzweigten Alkylkette von C₄ bis C₁₂; und/oder
(iii) einer Länge einer verzweigten Alkylkette von C₄ bis C₁₂ und einer Gesamtkopfgruppengröße von wenigstens 4 Ångström;
wobei die Zusammensetzung durch einen pH-Wert von 2,0 bis 4,5 **gekennzeichnet** ist;
wobei es sich bei der organischen Säure um Pyroglutaminsäure handelt; und
wobei die Zusammensetzung C₈-Alkylglycerylsulfonat (C8-AGS) umfasst.

2. Verwendung gemäß Anspruch 1, wobei die Krankheiten oder Infektionen atopische Dermatitis, Windeldermatitis, Impetigo, Blepharitis, Mastitis und oropharyngeale Candidose (OPC) umfassen.

3. Verwendung gemäß Anspruch 1, wobei die antimikrobielle Zusammensetzung wenigstens 0,5% C8-Alkylglycerylsulfonat (C8-AGS) umfasst.

4. Verwendung gemäß einem der vorstehenden Ansprüche, wobei die antimikrobielle Zusammensetzung weiterhin ein nichtionisches Mittel umfasst.

5. Verwendung gemäß Anspruch 4, wobei das nichtionische Mittel aus der Gruppe ausgewählt ist, die aus 1-(2-Ethylhexyl)glycerinether, Octylglycerinether, 2-(2-Ethylhexyloxy)octyloxypropanol, 1-(2-Ethylhexyloxy)-ethanol, Octyloxyethanol, 1,2-Hexylendiol, 1,2-Cyclohexandimethanol, Isopropylglycerinether und Kombinationen davon besteht.

6. Verwendung gemäß Anspruch 5, wobei das nichtionische Mittel 1-(2-Ethylhexyl)glycerinether umfasst.

7. Verwendung gemäß einem der Ansprüche 4 bis 6, wobei das nichtionische Mittel in der antimikrobiellen Zusammensetzung in einer Menge von 0,1 bis 10 Gew.-% der gesamten Zusammensetzung vorhanden ist.

8. Verwendung gemäß Anspruch 1, wobei die antimikrobielle Zusammensetzung Folgendes umfasst:
wenigstens 2,0 Gew.-% der gesamten Zusammensetzung an Pyroglutaminsäure; und
wenigstens 1,25 Gew.-% der gesamten Zusammensetzung an dem anionischen Tensid C₈-Alkylglycerylsulfonat.

9. Verwendung gemäß einem der Ansprüche 1 bis 8, wobei das Medikament topisch angewendet wird.

10. Verwendung gemäß einem der Ansprüche 1 bis 9, wobei die antimikrobielle Zusammensetzung mit Hilfe einer Waschflüssigkeit, eines Sprays, einer Einweichungslösung oder Tuchs angewendet wird.

11. Verwendung gemäß einem der Ansprüche 1 bis 10, wobei das Medikament als Imprägnat oder Beschichtung eines Körperpflegeprodukts angewendet wird.

12. Verwendung gemäß einem der Ansprüche 1 bis 10, wobei die Zusammensetzung in einer Menge angewendet wird, die die Desinfektion eines medizinischen Geräts zur Verwendung an einem Patienten bewirkt.

## Revendications

1. Utilisation d'une composition antimicrobienne pour la préparation d'un médicament destiné au traitement ou à la prévention de maladies ou d'infections provoquées par au moins un microbe choisi dans le groupe comprenant : *C. albicans,* les entérocoques résistants à la vancomycine (VRE), *P. aeruginosa, S. marcescens, S. aureus* et *Staphylococcus aureus* résistant à la méthicilline (MRSA), **caractérisée en ce que** ladite composition antimicrobienne comprend en poids :
a) de 0,2 % à 70 % d'un acide organique ; et
b) de 0,1 % à 40 % d'un mélange de surfactants anioniques ayant
(i) une longueur de chaîne alkyle linéaire de C₄ à C₁₂ et une taille totale de groupe de tête d'au moins 4 Angströms ; et/ou
(ii) une longueur de chaîne alkyle ramifiée de C₄ à C₁₂ ; et/ou
(iii) une longueur de chaîne alkyle ramifiée de C₄ à C₁₂ et une taille totale de groupe de tête d'au moins 4 Angströms ;
où ladite composition est **caractérisée par** un pH de 2,0 à 4,5 ;
où ledit acide organique est l'acide pyroglutamique ; et
où ladite composition comprend un sulfonate d'alkyl en C₈-glycéryle (C8-AGS).

2. Utilisation selon la revendication 1, où lesdites maladies ou infections comprennent : la dermite atopique, l'érythème fessier du nourrisson, l'impétigo, la blépharite, la mastite et la candidose oro-pharyngée (OPC).

3. Utilisation selon la revendication 1, où ladite composition antimicrobienne comprend au moins 0,5 % de sulfonate d'alkyl en C₈-glycéryle (C8-AGS).

4. Utilisation selon l'une quelconque des revendications précédentes, où la composition antimicrobienne comprend en outre un agent non ionique.

5. Utilisation selon la revendication 4, où ledit agent non ionique est choisi dans le groupe comprenant: le 1-(2-éthylhexyl)-glycérol éther, l'octyl-glycérol éther, le 2-(2-éthylhexyloxy)-octyloxy-propanol, le 1-(2-éthylhexyloxy)-éthanol, l'octyloxy-éthanol, le 1,2-hexylène-diol, le 1,2-cyclohexane-diméthanol, l'isopropyl-glycérol éther et des combinaisons de ceux-ci.

6. Utilisation selon la revendication 5, où ledit agent non ionique comprend du 1-(2-éthylhexyl)-glycérol éther.

7. Utilisation selon l'une quelconque des revendications 4 à 6, où ledit agent non ionique est présent dans ladite composition antimicrobienne dans une quantité de 0,1 % à 10 % en poids de la composition totale.

8. Utilisation selon la revendication 1, où ladite composition antimicrobienne comprend :
au moins 2,0 % en poids de la composition totale d'acide pyroglutamique ; et
au moins 1,25 % en poids de la composition totale du surfactant anionique sulfonate d'alkyl en C₈-glycéryle.

9. Utilisation selon l'une quelconque des revendications 1 à 8, où ledit médicament est appliqué par voie topique.

10. Utilisation selon l'une quelconque des revendications 1 à 9, où la composition antimicrobienne est appliquée en utilisant un savon liquide, un spray, un savon ou une lingette.

11. Utilisation selon l'une quelconque des revendications 1 à 10, où ledit médicament est appliqué sous la forme d'une imprégnation ou d'un enrobage d'un produit de soin personnel.

12. Utilisation selon l'une quelconque des revendications 1 à 10, où ladite composition est appliquée dans une quantité efficace pour désinfecter un dispositif médical pour une utilisation sur un patient.
